(19) **Europäisches Patentamt**
**European Patent Office**
**Office européen des brevets**

(11) **EP 2 208 465 A1**

(12) **EUROPEAN PATENT APPLICATION**
published in accordance with Art. 153(4) EPC

(43) Date of publication:
**21.07.2010 Bulletin 2010/29**

(21) Application number: **08846223.9**

(22) Date of filing: **28.10.2008**

(51) Int Cl.:
**A61B 8/08** (2006.01)

(86) International application number:
**PCT/JP2008/069507**

(87) International publication number:
**WO 2009/060751 (14.05.2009 Gazette 2009/20)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **09.11.2007 JP 2007292622**

(71) Applicant: **Hitachi Medical Corporation Chiyoda-ku Tokyo 101-0021 (JP)**

(72) Inventor: **CHONO, Tomoaki Tokyo 101-0021 (JP)**

(74) Representative: **Strehl Schübel-Hopf & Partner Maximilianstrasse 54 80538 München (DE)**

(54) **ULTRASONOGRAPHIC DEVICE, OPERATION METHOD THEREOF, AND ULTRASONOGRAM DIAGNOSIS PROGRAM**

(57)   An ultrasonic diagnostic apparatus including an ultrasonic probe for transmitting/receiving an ultrasonic wave to/from an examinee, an uniformity calculator for calculating parameters of a probability density function relating to uniformity of a biological tissue of the examinee from a probability distribution of amplitude values obtained at plural measurement points in a region of interest of an ultrasonic signal based on a reflected echo signal measured by the ultrasonic probe, an image generator for generating a tissue uniformity image gradated on the basis of the calculated parameters, and a display unit for displaying the generated tissue uniformity image.

FIG. 3

**Description**

Technical Field

The present invention relates to an ultrasonic diagnostic apparatus, a method of operating the same and an ultrasonic diagnostic program, and particularly to a technique of quantifying uniformity of an examinee's tissue and displaying an image thereof.

Background Art

An examiner such as a doctor, a clinical laboratory technologist or the like observes the movement, shape, characteristics, etc. of a target tissue in diagnosis using an ultrasonic diagnostic apparatus. The movement and shape of the tissue can be calculated in terms of physical quantities such as speed, length, etc. However, the characteristics reflect the tissue structure of a target, and thus it is difficult to directly quantitatively analyze the tissue structure due to limitation of resolution of ultrasonic pulses used for a medical ultrasonic diagnostic apparatus. Accordingly, it is generally calculated on the basis of the property of scattering wave of ultrasonic wave.

For example, an interference pattern of backscattering waves is called as a speckle, and the property thereof reflects the tissue structure. An examiner empirically distinguishes speckle patterns which are different among tissues and applies it to diagnosis. Various techniques for quantifying such qualitative diagnosis have been developed, and there has been proposed a method of paying attention to statistical properties of the amplitude values of speckles and utilizing that the amplitude probability is conformed with a probability density function corresponding to a tissue structure.

For example, Patent Document 1 describes a method of utilizing that the amplitude probability of scattering waves from a uniform tissue such as a normal liver or the like is conformed with a Rayleigh distribution and quantifying the difference between a normal tissue and an abnormal tissue on the basis of an error obtained by numerically expressing a deviation from the Rayleigh distribution.

Patent Document 1: JP-A-2003-61964

Disclosure of the Invention

Problem to be Solved by the Invention

However, in the technique described in the Patent Document 1, in some cases, it is impossible to properly express the degree of abnormality in a process that a normal tissue is gradually shifting to a lesion and thus the uniformity thereof is being lost.

That is, the Rayleigh distribution is a model for a uniform tissue. Therefore, there is a risk that an error obtained when the amplitude probability of the scattering wave is deviated from the Rayleigh distribution does not directly represent the degree of abnormality of the tissue. Accordingly, even when abnormality of the tissue can be represented by the deviation from the Rayleigh distribution, it may be impossible to represent how degree the uniformity is lost and how degree the tissue is abnormal.

An object of the present invention is to properly quantify and image uniformity of a biological tissue of an examinee on the basis of a probability distribution of amplitude values of an ultrasonic signal.

Means of Solving the Problem

An ultrasonic diagnostic apparatus according to the present invention is characterized by comprising: an ultrasonic probe for transmitting/receiving an ultrasonic wave to/from an examinee; an uniformity calculator for calculating parameters of a probability density function relating to uniformity of a biological tissue of the examinee from a probability distribution of amplitude values obtained at plural measurement points in a region of interest of an ultrasonic signal based on a reflected echo signal measured by the ultrasonic probe; an image generator for generating a tissue uniformity image gradated on the basis of the calculated parameters; and a display unit for displaying the generated tissue uniformity image.

A method by which an ultrasonic diagnostic apparatus functions according to the present invention is characterized by comprising: a step of generating a relative frequency distribution of amplitude values at plural measurement points in a region of interest of an ultrasonic signal based on a reflected echo signal which is obtained by transmitting/receiving an ultrasonic wave to/from an examinee; a step of calculating parameters of a probability density function having the parameters concerned, the parameters being related to uniformity of a biological tissue of the examinee, on the basis of a statistical moment obtained from the generated relative frequency distribution; a step of executing each of the steps while moving the region of interest; a step of generating a tissue uniformity image gradated on the basis of the calculated parameters in each region of interest; a step of generating a tissue uniformity image gradated on the basis of the parameters calculated in each region of interest; and a step of displaying the generated tissue uniformity image on display means.

An ultrasonic diagnostic program is characterized by making a computer execute: a procedure of generating a relative frequency distribution of amplitude values at plural measurement points in a region of interest of an ultrasonic signal based on a reflected echo signal which is obtained by transmitting/receiving an ultrasonic wave to/from an examinee; a procedure of calculating parameters of a probability density function having the parameters concerned, the parameters being related to uniformity of a biological tissue of the examinee, on the basis of a statistical moment obtained from the generated relative frequency distribution; a procedure of executing each of the steps while moving the region of interest; a procedure of generating a tissue uniformity image gradated on the basis of the calculated parameters in each region of interest; and a procedure of displaying the generated tissue uniformity image on display means.

Effect of the Invention

According to the present invention, the uniformity of the biological tissue of the examinee can be properly quantified and imaged on the basis of the probability distribution of the amplitude values of the ultrasonic signal.

Brief Description of the Drawings

[Fig. 1] Fig. 1 is a block diagram showing the construction of an ultrasonic diagnostic apparatus according to an embodiment.
[Fig. 2] Fig. 2 is a flowchart showing the processing flow according to a first embodiment.
[Fig. 3] Fig. 3 is a diagram showing creation of a tissue uniformity image of the first embodiment;
[Fig. 4] Fig. 4 is a diagram showing an actually measured relative frequency distribution and an estimated probability density function having high goodness-of-fit.
[Fig. 5] Fig. 5 is a diagram showing an actually measured relative frequency distribution and an estimated probability density function having low goodness-of-fit.
[Fig. 6] Fig. 6 is a diagram showing a method of setting ROI to calculate uniformity parameters.
[Fig. 7] Fig. 7 is a flowchart showing the processing flow of a second embodiment.
[Fig. 8] Fig. 8 is a diagram showing creation of a tissue uniformity image according to the second embodiment.
[Fig. 9] Fig. 9 is a flowchart showing the processing flow of a third embodiment.
[Fig. 10] Fig. 10 is a diagram showing creation of a tissue uniformity image according to the third embodiment.
[Fig. 11] Fig. 11 is a diagram showing a screen display of uniformity parameter values at any places.
[Fig. 12] Fig. 12 is a diagram showing an application example to an infarcted site in a heart four-chamber view display.
[Fig. 13] Fig. 13 is a diagram showing an application example to an infarcted site in a heart short-axis view display.
[Fig. 14] Fig. 14 is a diagram showing an application example to a liver tumor.
[Fig. 15] Fig. 15 is a diagram showing an application example to a mammary tumor.

Description of Reference Numerals

10 ultrasonic diagnostic apparatus, 12 ultrasonic probe, 14 RF signal generator, 16 ultrasonic image generator, 18 calculator, 18a uniformity calculator, 18b significance probability calculator, 20 uniformity image generator, 22 display image generator, 24 controller, 26 operation unit, 28 storage device, 30 display unit

Best Modes for carrying out the Invention

Embodiments of an ultrasonic diagnostic apparatus to which the present invention is applied will be described. In the following description, the same function parts are represented by the same reference numerals, and the duplicative description thereof is omitted.

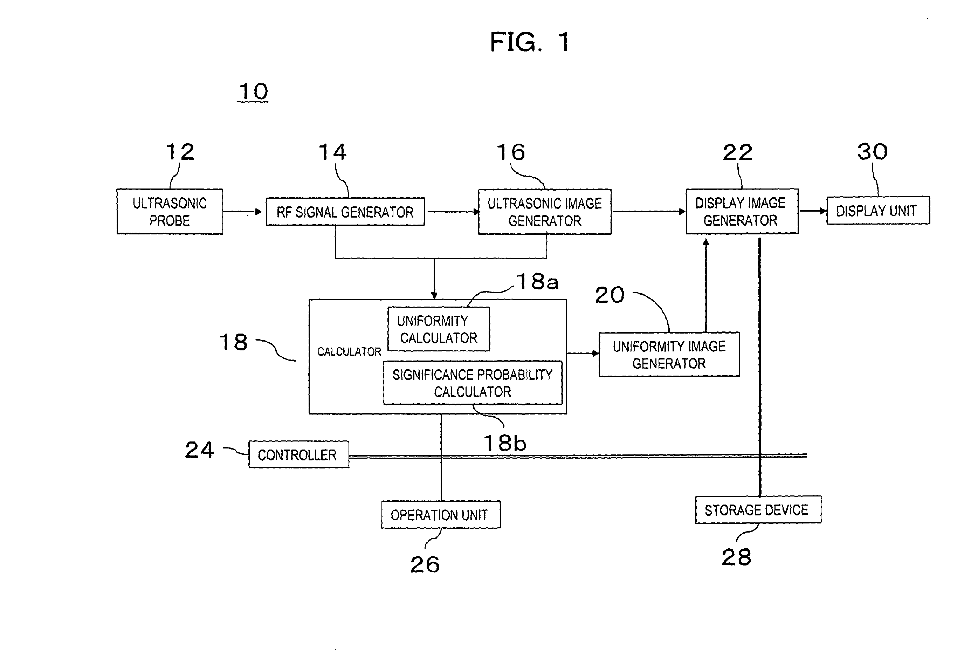

Fig. 1 is a block diagram showing the ultrasonic diagnostic apparatus according to an embodiment. As shown in Fig. 1, the ultrasonic diagnostic apparatus 10 includes an ultrasonic probe 12 for transmitting/receiving an ultrasonic wave, an RF signal generator 14 for generating an RF signal from a reception wave, and an ultrasonic image generator 16 for generating M, B, 3D mode images from the RF signal.
The ultrasonic diagnostic apparatus 10 further includes a calculator 18 which receives an ultrasonic signal output from the RF signal generator 14 or the ultrasonic image generator 16 to calculate parameters of a probability density function and performs a goodness-of-fit test, and a uniformity image generator 20 for generating a tissue uniformity image gradated on the basis of the calculated parameters. The ultrasonic signal output from the RF signal generator 14 is an RF signal, and the ultrasonic signal output from the ultrasonic image generator 16 is a signal obtained by subjecting an RF signal to logarithmic compression processing.

The ultrasonic diagnostic apparatus 10 further includes a display image generator 22 for associating the ultrasonic image generated in the ultrasonic image generator 16 with the tissue uniformity image generated in the uniformity image generator 20 to generate a display image, a controller 24 for controlling an instruction and a timing for the overall calculation, an operation unit 26 through which an examiner operates the device by using input equipment, a storage device 28 in which signal data, a control program, a calculation program, etc, are stored, and a display unit 30 for displaying an image.

The ultrasonic probe 12 transmits/receives an ultrasonic wave to/from an examinee, and it has a shape such as a sector type, a linear type, a convex type or the like. In addition, various well-known ultrasonic probes may be applied. The RF signal generator 14 receives a reflected echo signal (reception acoustic wave) which is received and converted to an electrical signal by plural oscillators of the ultrasonic probe 12, and converts the reflected echo signal to an RF signal through a phasing adder.

The ultrasonic image generator 16 receives the RF signal converted in the RF signal generator 14, and converts the RF signal to an M mode image, a B mode image and a 3-D image (hereinafter referred to as proper ultrasonic image) through a logarithmic amplifier, an envelop curve detector, an A/D converter and a scan converter.

The calculator 18 comprises a uniformity calculator 18a which receives the RF signal from the RF signal generator 14 or receives an image signal from the ultrasonic image generator 16 and calculates parameters of a probability density function from a statistical property of a probability distribution of amplitude values at plural measurement points in a set region of interest (hereinafter referred to as ROI), and a significance probability calculator 18b for executing a goodness-of-fit test between the probability density function defined by the calculated parameters and an observation distribution to calculate a significance probability.

The uniformity image generator 20 subjects the parameters calculated in the uniformity calculator 18a to scaling, and generates a tissue uniformity image which is gradated by grey- scale or pseudocolor. This imaging processing is executed on only data having a significance probability which is not less than some significant level as a preset threshold value.

The display image generator 22 directly superposes the tissue uniformity image generated in the uniformity image generator 20 on the ultrasonic image, or subjects the tissue uniformity image to translucent processing and then superposes the processed tissue uniformity image on the ultrasonic image, thereby generating a display image.

The controller 24 outputs an instruction to each signal processor to control the timing thereof. The operating unit 26 is input equipment such as a keyboard, a mouse, a track ball or the like disposed on the diagnosis device, and the examiner uses it to adjust the image quality, instruct measurement, input information, etc.

The storage device 28 stores signal data, control programs, calculation programs, etc. Furthermore, calculation parameters such as the preset probability density function, significant level, the size of ROI, etc. are stored in the storage device 28. The display unit 30 is display equipment such as a display or the like, and it displays an image generated in the display image generator 22, etc.

The uniformity calculator 18a, the significance probability calculator 18b and the uniformity image generator 20 which are feature portions of the present invention will be described together with the operation of the ultrasonic diagnostic apparatus by using embodiments.

Embodiment 1

An embodiment 1 is an embodiment when a tissue uniformity image is generated and displayed while superposed on a B mode image. First, an examiner operates the operation unit 26 to start measurement of the tissue characteristics of an examinee, that is, the uniformity image of the tissue while changing to a display mode.

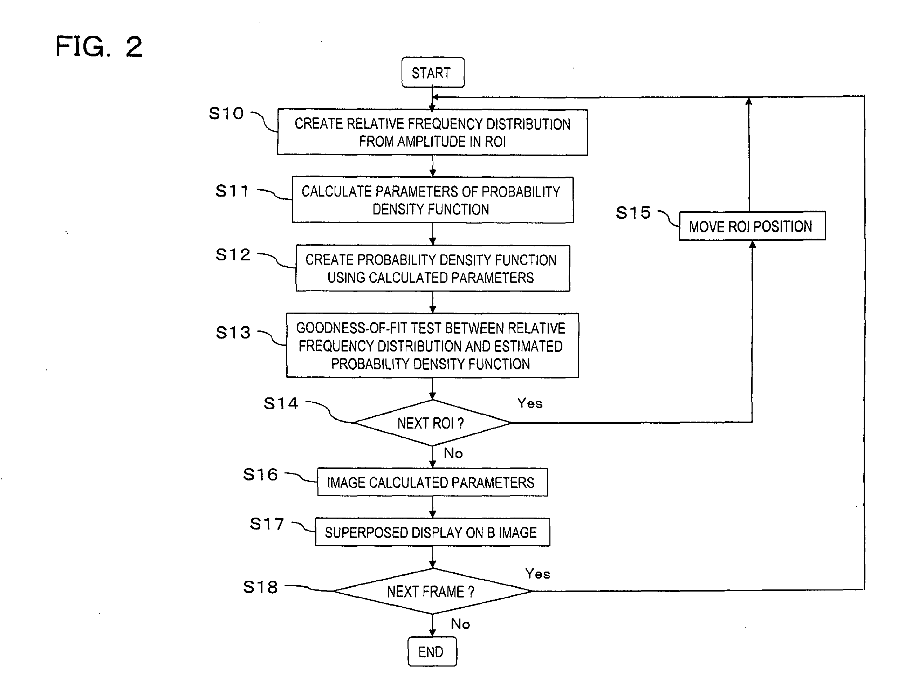

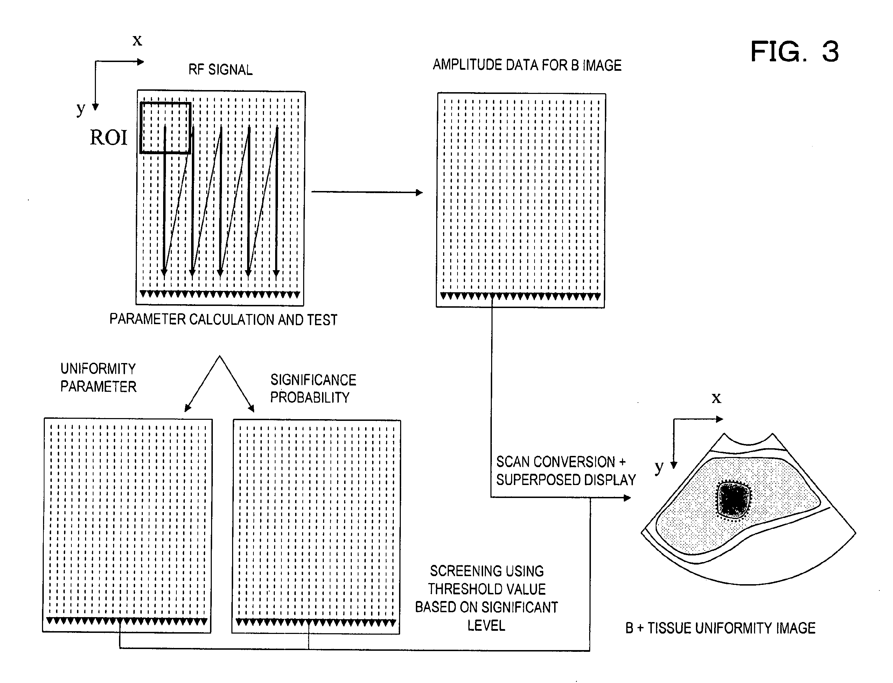

Fig. 2 is a flowchart showing the processing flow of the first embodiment. First, ROI for calculating a statistic property is set for an RF signal which is obtained by transmitting/receiving an ultrasonic wave to/from the examinee as shown at the upper left side of Fig. 3. Amplitude values at plural measurement points located on a scanning line surrounded by the ROI are obtained, and a relative frequency distribution is created by using the obtained amplitude values of the ultrasonic signal (S10).

The size of ROI which affects the resolution and the estimation precision of the uniformity of the tissue is properly adjusted. ROI may be set from the operation unit 26 by the examiner, or it may be automatically set to a recommendation value in advance. The description will be made on the condition that the shape of ROI is a rectangle, however, it may be modified to a circle or a sector form. Time-averaging processing of the signal in ROI may be added to reduce white noise.

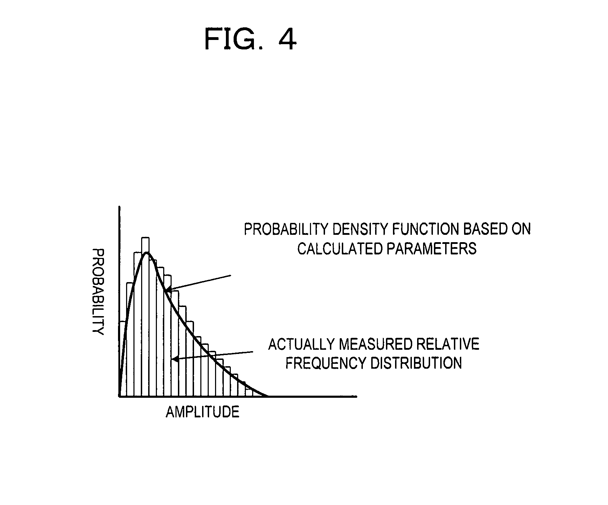

The relative frequency distribution created here is as shown in Fig. 4, for example. Subsequently, the parameters of the probability density function are calculated from the statistic property of the relative frequency distribution (S11).

Here, many models have been proposed for the probability density function of amplitude values of ultrasonic scattering wave, and K-distribution, generalized K-distribution, Homodyned K-distribution, Nakagami distribution, generalized Nakagami distribution, Gamma distribution, generalized Gamma distribution, Rice distribution, generalized Rice distribution, Weibull distribution, lognormal distribution, etc. are known as models having non-uniformity as a parameter. These distribution functions are prepared in the storage device 28 in advance, and probability density setting means which can select a distribution corresponding to a biological tissue is provided. The examiner may select a function, or a function to be recommended may be set by a device.

A method of calculating the parameters in the uniformity calculator 18a will be described by applying to an example using the K-distribution. The K-distribution is represented by an expression (1).

[Mathematical expression 1]

$$p(A) = \frac{2c}{\Gamma(a)}\left(\frac{cA}{2}\right)^{\alpha} K_{\alpha-1}(cA) \quad ; \quad c = \frac{\sqrt{2\alpha}}{\phi}(A \geq 0) \tag{1}$$

Here, p represents a function representing the probability that amplitude A appears, $\Gamma$ represents gamma function, k represents modified Bessel function of the second kind. $\phi$ represents a scale parameter, and $\alpha$ represents a shape parameter. $\phi$ represents a parameter dependent on signal intensity. Furthermore, $\alpha$ is called as an effective scatterer number (the number of scatterers contributing to amplitude in the resolution of ultrasonic pulses), and it is a parameter representing the uniformity of the scatterer. For $\alpha$ of 10 or more, the scatterer becomes uniform (approximate to the Rayleigh distribution), and it becomes non-uniform as $\alpha$ decreases. That is, the calculation of $\alpha$ means estimation of the uniformity of a tissue.

A method of combining statistical quantities or using a maximum likelihood estimate method has been proposed as a method of calculating $\alpha$. For example, according to the method using the statistical quantities, $\alpha$ and $\phi$ are determined as follows (Non-patent document: Dutt V, Greenleaf JF.K distribution model of ultrasound speckle: fractional order SNRs and log compression variance. IEEE Proc. Ultrasonics Symposium 1995; 2: 1375-1378)

[Mathematic expression 2]

$$\mu_n = (2\phi^2)^{\frac{n}{2}} \frac{\Gamma(1+n/2)\Gamma(\alpha+n/2)}{\alpha^{n/2}\Gamma(\alpha)} \tag{2}$$

[Mathematic expression 3]

$$\frac{\mu_n}{\sqrt{\mu_{2n}-\mu_n^2}} = \left[\frac{\Gamma(1+n)\Gamma(\alpha+n)}{\Gamma(1+n/2)\Gamma(\alpha+n/2)} - 1\right]^{-\frac{1}{2}} \tag{3}$$

Here, $\mu_n$ represents an n-order moment statistical quantity determined from the relative frequency distribution. By substituting this $\mu_n$ in the mathematical expressions (2) and (3), $\phi$ and $\alpha$ are calculated. $\alpha$ is a parameter which is not dependent on the signal intensity, and it potentially serves as an absolute parameter for estimating uniformity.

When $\phi$ and $\alpha$ are calculated, the K-distribution as a probability density function is created from the mathematic expression (1) by using $\phi$ and $\alpha$ (S12). By substituting $\phi$ and $\alpha$ into the mathematical expression (1), the K-distribution is uniquely determined.

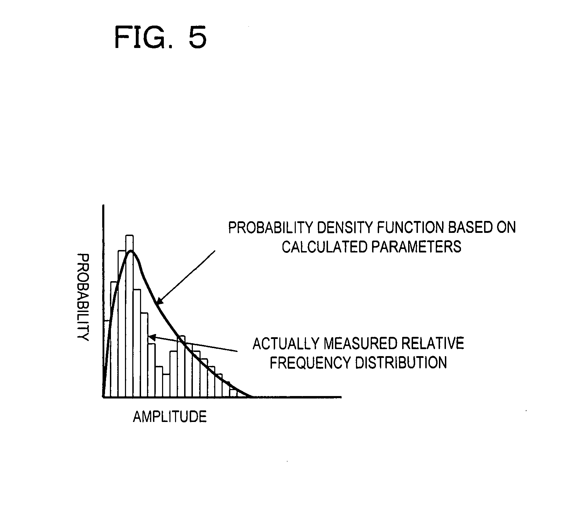

For example, it is assumed that the determined K-distribution function is represented by a solid-line curve of Fig. 4. In this case, the observed relative frequency distribution and the calculated probability density function are excellently coincident with each other. Conversely, when parameters for a relative frequency distribution of Fig. 5 are calculated and a probability density function indicated by a solid line is created, it is found that the observed relative frequency distribution and the calculated probability density function are not so coincident with each other. That is, in this case, it is indicated that the reliability of the calculated parameters is low.

Therefore, the good-of-fit between the actually measured relative frequency distribution and the calculated probability density function is tested by the significance probability calculator 18b (S13). This means that the shapes of these two distributions are compared with each other. In the good-of-fit test, when there is some calculated theoretical probability density function, it can be checked whether the actually observed frequency distribution is substantially coincident with the theoretical probability density function.

That is, a null hypothesis is "an observed distribution is a set probability distribution", and it may be determined whether this is adopted or not. A chi-square test statistical quantity is determined from the actually measured relative frequency distribution and the estimated probability density function to calculate a significance probability. When the significance probability is higher than a preset significant level, the null hypothesis is adopted, and thus "it is not necessarily said that the observed distribution is not the set probability distribution". Conversely, when the significance probability is lower than the significant level, the null hypothesis is rejected, and it is said that the observed distribution is not the set probability distribution". In the latter case, the probability density function defined by the calculated parameters is not fit to the actually measured relative frequency distribution, and thus it means that the reliability of the parameters is low.

The chi-square test statistical quantity is determined according to the mathematical expression (4).

[Mathematical expression 4]

$$\chi^2 = \sum_{i=1}^{m} \frac{(O_i - E_i)^2}{E_i} \qquad (4)$$

m represents the number of categories, O represents the actually measured relative frequency, and E represents the estimated amplitude probability. When the square value of x of the mathematical expression (4) is small, the error between the actually measured relative frequency distribution and the estimated probability density function is small. Conversely, when the square value of x is large, the error is large, and the probability that the actually measured relative frequency distribution is not fit to the estimated probability density function is high. Here, since the square value of x is conformed with the square distribution of x, and thus the upper-side probability of the square distribution of x is calculated by using the square value of x determined in the mathematical expression (4). This is the significance probability. In this case, when the actually measured relative frequency distribution and the probability density function are fit to each other, the significance probability represents the probability that such an error is found out. Accordingly, whether the error appearing with the above probability is permitted or not is determined by screening using the threshold value based on the significant level.

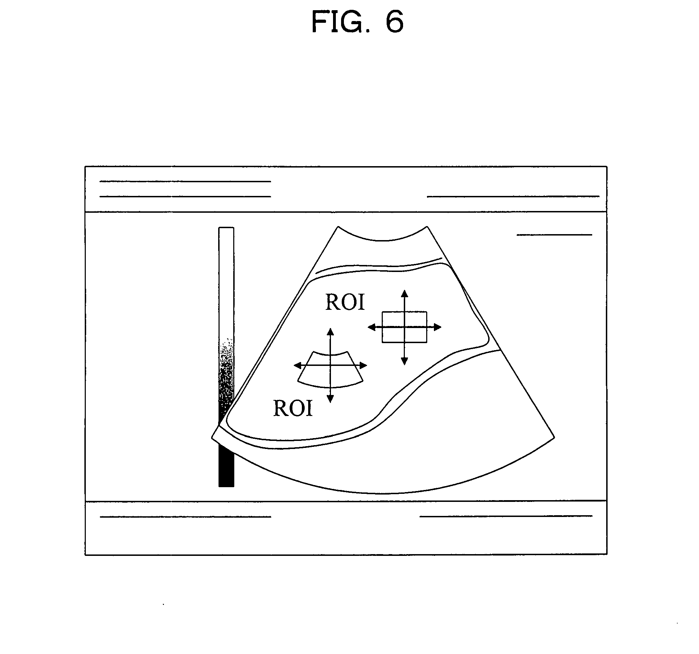

The processing of S10 to S13 is executed on ROIs of the overall image (S14, S15). That is, the processing of S10 to S13 is repeated while moving to next ROI along the scanning line as indicated by an arrow at the upper left diagram of Fig. 3 (S15). When only a local of the image is observed, ROI can be set to any local as shown in Fig. 6, and ROI for obtaining the amplitude value is set in the local ROI to perform the calculation.

When the overall processing on the signal is finished, the uniformity parameters and the data of the significance probability on all the scanning lines which constitute the B mode image as shown in the lower left diagram of Fig. 3. Here, when the significance probability is lower than the significant level, it is estimated that a tissue boundary or a structure such as a blood vessel or the like is a non-echo area.

Accordingly, in order to remove such an area from an imaged area, imaging is executed on only the data above the set significant level in the calculated parameters (S16). Accordingly, it is possible to perform imaging on only the parameters which are high in reliability.

The tissue uniformity image generated by the uniformity image generator 20 is an image which is gradated by subjecting the calculated parameters to scaling from perfect uniformity to non-uniformity and also converting them to gray scale or pseudocolor. For example, in the case of the K-distribution, pseudocolor or gray scale display is performed so that the color is set to red color for uniformity $\alpha > 10$ and the color is gradually varied to blue color as $\alpha$ shifts to 0.

Furthermore, the conventional processing for B-mode display is also executed in the ultrasonic image generator 16. In the display image generator 22, the tissue uniformity image generated in S16 and the B-mode image are associated with each other. Specifically, the tissue uniformity image is displayed while superposed on the ultrasonic image, or the tissue uniformity image is subjected to translucent processing and then displayed while superposed on the ultrasonic image (S17). An image obtained by subjecting the tissue uniformity image to the translucent processing and then displaying the processed tissue uniformity image while the tissue uniformity image is superposed on the B-mode image is as shown in the lower right diagram of Fig. 3. As described above, by superposing the tissue uniformity image on the B-mode image and displaying the superposed tissue uniformity image, the tissue characteristics can be observed while grasping the positional relationship of the tissue, and it can be displayed to be intuitively easily viewable.

By sequentially applying the processing of S10 to S17 to the next frame (S18), the observation of the tissue characteristics on a moving image can be performed. The probability density function which can be modeled is different among sites, and thus the tissue characteristics can be observed while the probability density function prepared in the storage device 28 is changed as needed so that an image which makes the tissue characteristics easily viewable can be obtained.

Furthermore, the significant level can be variably adjusted so as to obtain an image which is easily viewable because a structure portion such as a blood vessel or the like and a non-echo area are removed. As the significant level is lower, parameters having lower reliability are permitted. However, an image is hardly viewable because the boundary of a structure or parameters of a non-echo area are imaged. Conversely, when the significant level is excessively high, only parameters of a strictly fitted distribution are displayed, and thus it is necessary to properly adjust the significant level.

With respect to the size of ROI, as it is larger, the reliability of estimation is enhanced, however, the resolution is lowered. Conversely, when it is small, the number of samples is small and thus the reliability of estimation is lowered. Therefore, the size of ROI is adjusted so as to obtain an easily-viewable image. The above settings can be adjusted by the input equipment of the operation unit 26 as needed.

In this embodiment, the RF signal generated in the RF signal generator 14 is input to the calculator 18. However, the ultrasonic image generated in the ultrasonic image generator 16 may be input. In this case, the logarithmic compression processing is executed by the ultrasonic image generator 16, and thus it has a statistic property different from the RF signal. Therefore, a logarithmically compressed probability density function is prepared in the storage device 28, and the function is switched to a logarithm-compression adaptable function when an image after the logarithmic compression is input.

In order to quantify a process that a tissue is shifting to a lesion, it is necessary to estimate the gradually varying property without being dependent on the quality of the signal, and it is desired that the property concerned can be represented by an absolute numerical value.

According to this embodiment, the parameters representing the tissue characteristics can be determined from the distribution of the amplitude values of the ultrasonic signal without being dependent on the quality of the signal by using the probability density function (K-distribution) having the uniformity of a biological tissue as parameters, and the uniformity which is gradually varied in the process that the biological tissue shifts to a lesion can be properly qualified.

Furthermore, with respect to areas in which the amplitude probability of scattering wave is not conformed with the probability density function, such as a tissue boundary, a blood vessel, etc., it is necessary to determine that the reliability of the qualified value is low. According to this embodiment, the reliability of the calculated parameters can be estimated by testing how degree the observed relative frequency distribution is fit to the probability density function defined by the calculated parameters. Imaging can be performed at only positions at which the parameters have high reliability. Therefore, the examiner can easily discriminate the degree of abnormality in the process that the biological tissue is shifting to a lesion.

Embodiment 2

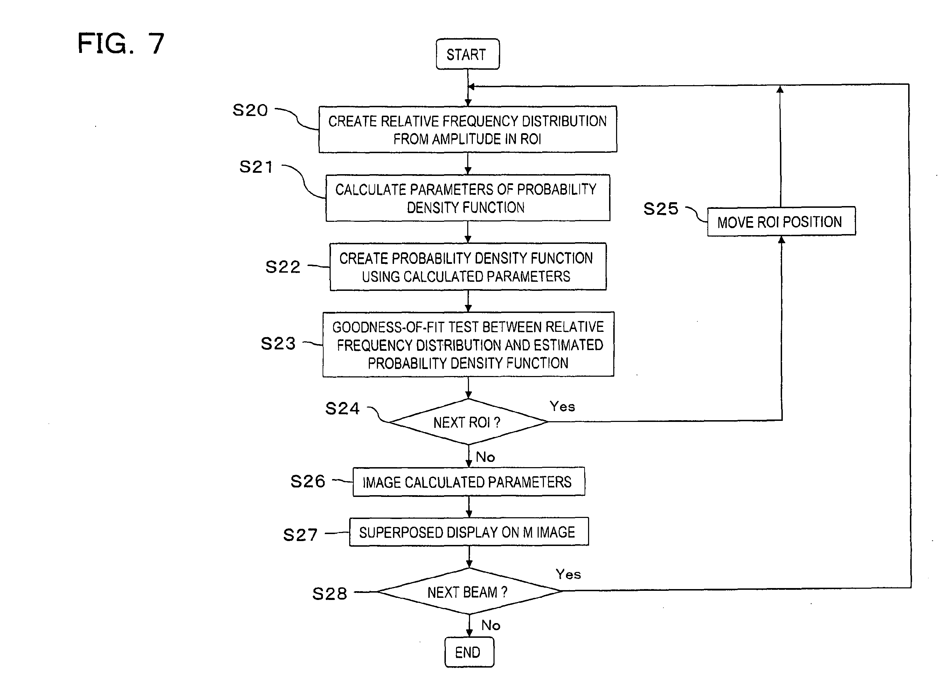

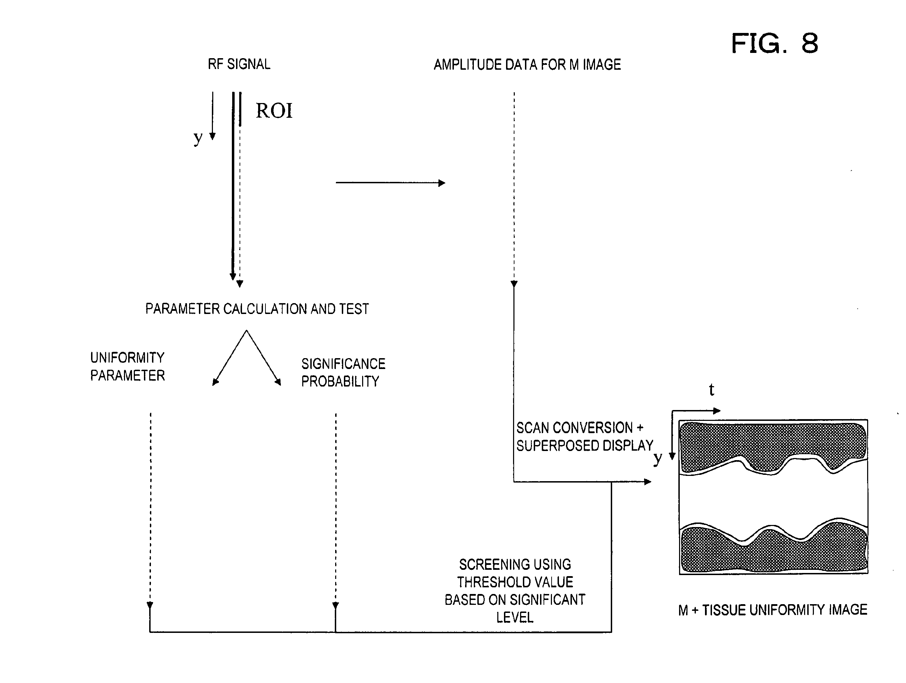

A second embodiment is an embodiment in which a tissue uniformity image is generated and displayed while superposed on an M-mode image. Fig. 7 is a flowchart showing the processing flow of the second embodiment. The same premised processing as the embodiment 1 is executed, and then amplitude values of linear ROI on the scanning line are extracted as shown in an upper left diagram of Fig. 8 to create a relative frequency distribution (S20). The processing from S21 to S23 is the same as the processing from S11 to S13 of the embodiment 1.

When the calculator 18 executes the processing of S20 to S23 while ROI is moved on the scanning line (S24, S25), uniformity parameters and a significance probability for one line of the A mode are obtained as shown in a lower left diagram of Fig. 8. The uniformity parameters and the significance probability are screened by the threshold value based on the significance probability, and only the uniformity parameters having reliability are imaged (S26) and displayed while superposed on the M-mode image as shown in a lower right diagram of Fig. 8 (S27). When there is a next beam, the same processing is executed (S28).

By executing the processing as described above, the variation process of the tissue characteristics corresponding to the variation of motion at some position of a tissue can be easily observed as in the case of the first embodiment.

Embodiment 3

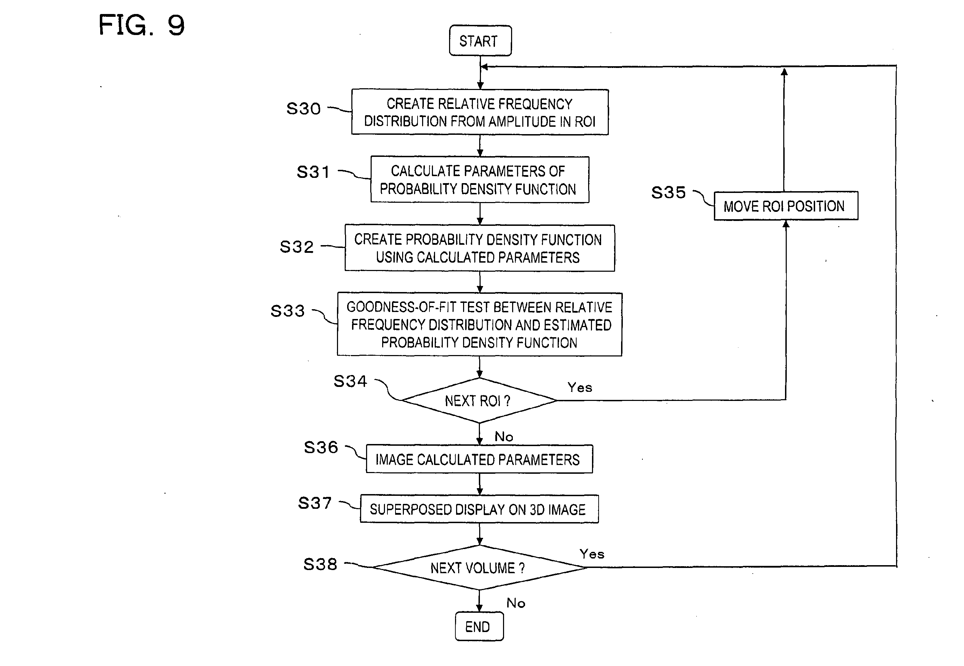

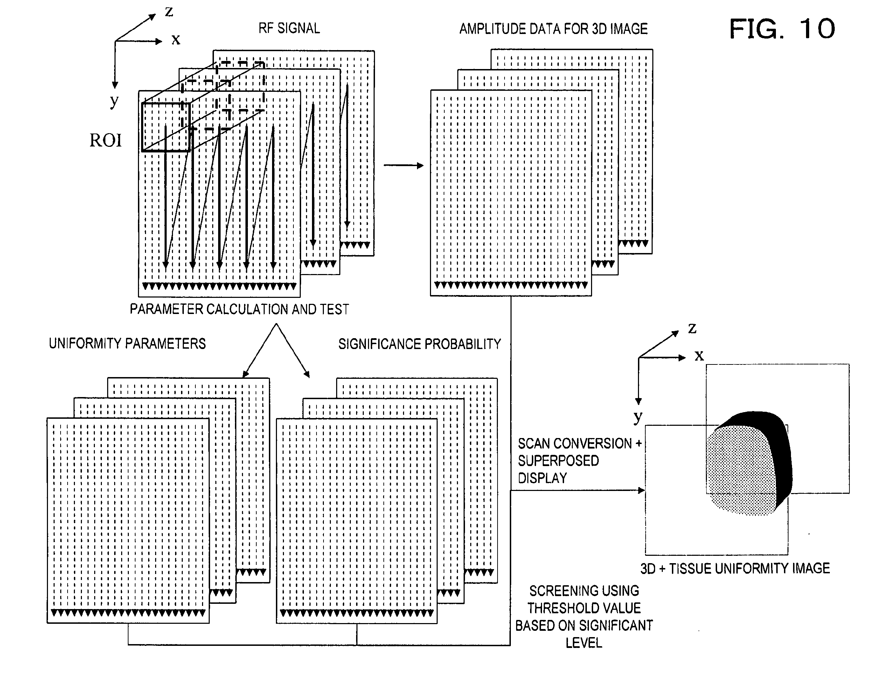

An embodiment 3 is an embodiment when a tissue uniformity image is generated and displayed while superposed on a 3D mode image. Fig. 9 is a flowchart of the processing flow of the third embodiment. As in the case of the first embodiment, after the premised condition is executed, a rectangular parallelepiped ROI is set so that a rectangle on an xy-plane is three-dimensionally expanded in a z-direction as shown in an upper left diagram of Fig. 10. Amplitude values contained in the rectangular parallelepiped ROI are obtained, and a relative frequency distribution is created by using the obtained amplitude values of the ultrasonic signal (S30). The processing from S31 to S33 is the same as the processing from S11 to S13 of the embodiment 1. The shape of ROI may be deformed to a sphere or sector.

When the calculator 18 repeats the processing from S30 to S33 while ROI is three-dimensionally moved on the scanning line (S34, S35), uniformity parameters and a significance probability on all the beams constituting a 3D image are determined as shown in a lower left diagram of Fig. 10. The uniformity parameters and the significance probability are screened by the threshold value based on the significant level. Therefore, only the reliable uniformity parameters are imaged (S36), and displayed while superposed on the 3D image as shown in a lower right diagram of Fig. 10 (S37). When there is a next volume (S38), the processing is likewise executed.

By executing the processing as described above, the property of the overall tissue can be three-dimensionally observed, and also the degree of abnormality in the process that the tissue is shifting to a lesion can be easily observed as in the case of the first embodiment.

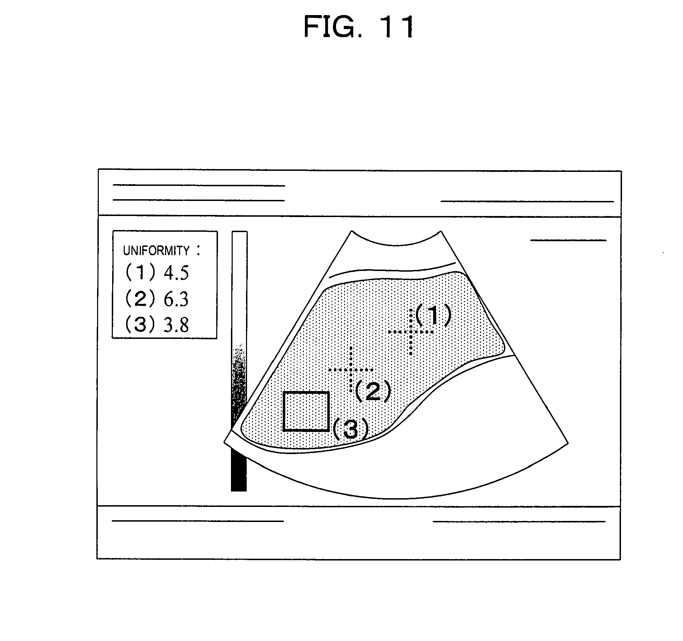

With respect to GUI of the display unit 30 of the embodiments 1 to 3, the displayed uniformity parameters can be recognized as numerical values as shown in Fig. 11. The following description is made by applying to the B mode image as an example. However, the same description is applicable to the M, 3D images. For example, as shown in Figs. 11(1) (2), a parameter value at a position at which a caliper is set is displayed on an image, whereby the difference in property between tissues can be measured, and thus this embodiment is applicable to the qualitative discrimination of an abnormal tissue.

Furthermore, as shown in Fig. 11(3), the statistical quantities of the parameters in ROI, for example, the average and the standard deviation are calculated and displayed, whereby the property can be easily understandably displayed.

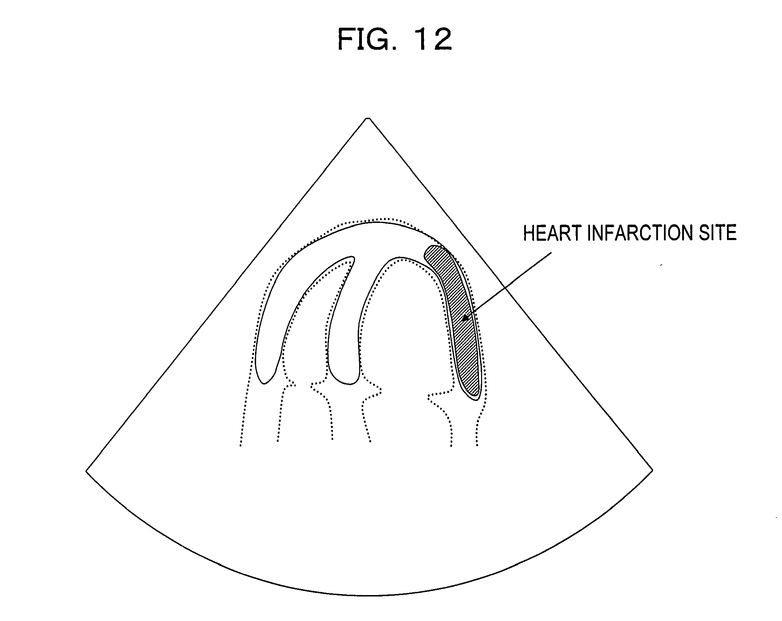

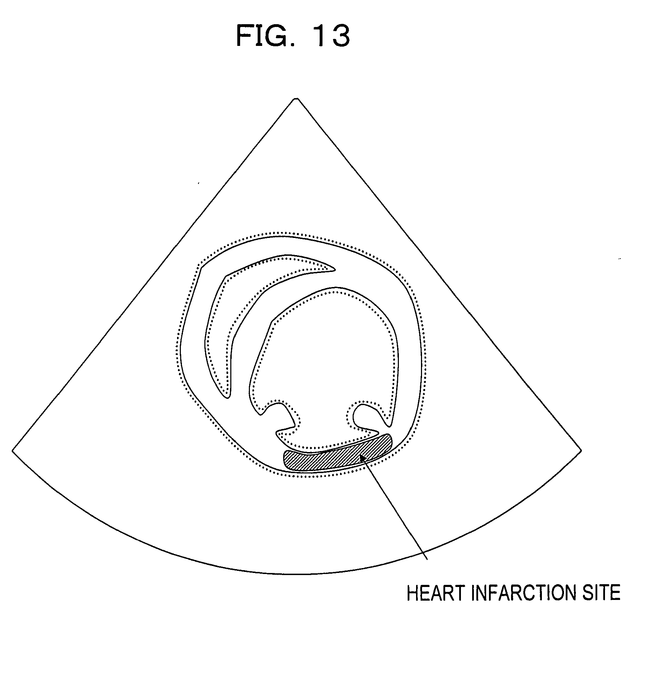

It is generally known that the amplitude probability of scattering waves from a biological soft tissue such as a heart muscle, a liver, a mammary gland or the like is conformed with some probability density function. A clinical application of the present invention will be described hereunder with reference to Fig. 12 and subsequent figures. Figs. 12 and 13 show a tomogram and a short-axis image of four chambers of a heart. A dotted line represents conventional images of a four-chamber image and a short-axis image which are displayed in the B mode. A solid line portion corresponds to a heart muscle portion, and scattering waves are dominant at this portion, so that the uniformity parameters are imaged at this area. Blood exists in the heart chamber, however, it provides no echo. Therefore, the reliability of the parameters is lowered, and these parameters are not imaged. Furthermore, when an infarcted site exists as represented by a hatched portion, it is displayed with a color different from that of the other heart muscles.

The infarcted site becomes necrotic because no blood is fed to the infarcted site. The uniformity thereof is different from that of normal heart muscles, and thus when an infarcted site or a lesion is imaged, the degree of the infarcted site or the lesion is displayed to be intuitively viewable.
Furthermore, upon observation on a moving picture, when there is a variation in uniformity of a heart muscle tissue due to beating of a heart muscle, the variation thereof can be observed as a timely color variation. Furthermore, actually, scattering waves cannot be received at some faraway positions from the probe due to attenuation of ultrasonic waves or artifact. In this case, it is determined by the test that the reliability of parameters is low, and thus this area is determined as an area which is not imaged.

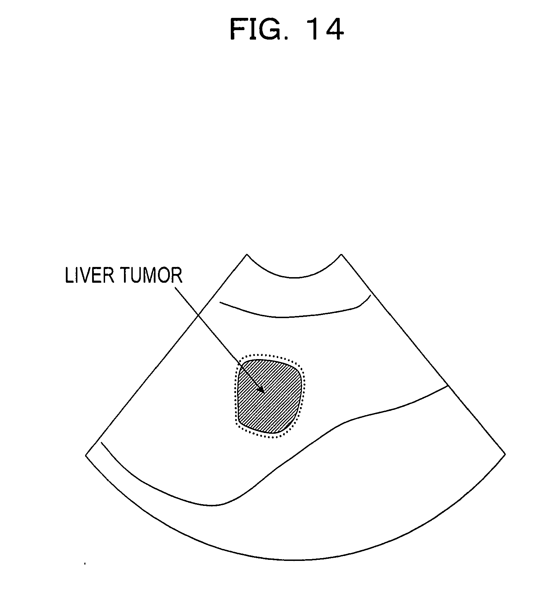

Fig. 14 shows an image in which a liver tumor is displayed. An area surrounded by a solid line is a liver parenchyma, and if it is normal, it is displayed with a color representing high uniformity. However, when it is a diffuse lesion such as liver cirrhosis or fatty liver, the uniformity thereof varies, and thus the overall liver parenchyma is displayed with a color representing non-uniformity.

Furthermore, when there exists a localized lesion such as a liver tumor represented by a hatched area, the color corresponding to the type is displayed. For example, in the case of hepatic cyst, it may provide no echo in some cases. In the case of hepatic cell carcinoma, the uniformity thereof is different in accordance with the process thereof or whether it is protopathic or metastatic. Therefore, the color corresponding to the situation described above is displayed.

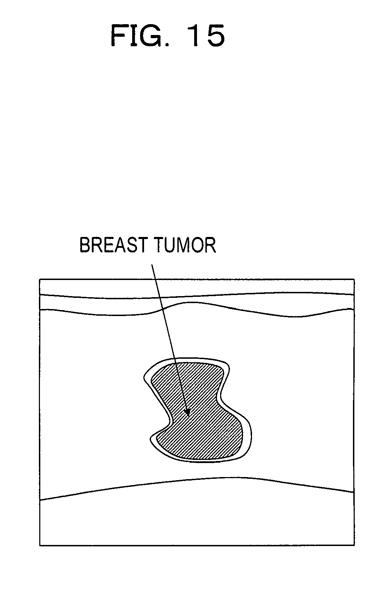

Fig. 15 shows an image in which a mammary tumor is displayed. In a normal mammary tumor, it is displayed with a color representing high uniformity. When a mammary tumor represented by a hatched area exists, it is displayed with the color corresponding to the type thereof. For example, in the case of cyst, it frequently provides no echo, and in the case of breast cancer, the range of an area representing uniformity is different in accordance whether it is infiltrative or not infiltrative. Furthermore, no scattering wave is obtained at a calcified site, and thus the reliability of the parameters is lowered, so that an indication of non-imaging or the like is displayed.

The preferable embodiments of the medical image display device according to the present invention have been described. However, the present invention is not limited to these embodiments. It is obvious that persons skilled in the art can make various kinds of alterations or modifications within the scope of the technical idea disclosed in this application, and it is understandable that they belong to the technical scope of the present invention.

**Claims**

1. An ultrasonic diagnostic apparatus, **characterized by** comprising:

   an ultrasonic probe configured to transmit/receive an ultrasonic wave to/from an examinee;
   an uniformity calculator configured to calculate parameters of a probability density function relating to uniformity of a biological tissue of the examinee from a probability distribution of amplitude values obtained at plural measurement points in a region of interest of an ultrasonic signal based on a reflected echo signal measured by the ultrasonic probe;
   an image generator configured to generate a tissue uniformity image gradated on the basis of the calculated parameters; and a display unit for displaying the generated tissue uniformity image.

2. The ultrasonic diagnostic apparatus according to claim 1, wherein the uniformity calculator applies a moment statistic quantity obtained from a relative frequency distribution of the amplitude values at the plural measurement points to the probability density function to calculate the parameters.

3. The ultrasonic diagnostic apparatus according to claim 1, further comprising significance probability calculating means configured to perform a goodness-of-fit test between a probability density function defined by the parameters calculated by the uniformity calculator and a relative frequency distribution of the amplitude values at the plural measurement points, thereby calculating a significance probability, wherein the image generator generates a tissue uniformity image on the basis of parameters which are contained in the calculated parameters and specified by the significance probability.

4. The ultrasonic diagnostic apparatus according to claim 1, further comprising a probability density function setting unit configured to selectively set plural probability density functions which are different in accordance with the type of a biological tissue.

5. The ultrasonic diagnostic apparatus according to claim 1, wherein the uniformity calculator calculates the parameters every region of plural regions of interests which are set for the ultrasonic signal, and the image generator generates the tissue uniformity image on the basis of the parameters calculated every region.

6. The ultrasonic diagnostic apparatus according to claim 1, wherein the ultrasonic signal is at least one of an ultrasonic wave RF signal generated by the RF signal generating means on the basis of the reflected echo signal and a signal obtained by performing logarithmic compression on the ultrasonic image generated by the ultrasonic image generating means on the basis of the ultrasonic RF signal.

7. The ultrasonic diagnostic apparatus according to claim 1, wherein the biological tissue of the examinee is a tissue for which an amplitude probability of an ultrasonic scattering wave from the tissue is modeled by a probability density function.

8. The ultrasonic diagnostic apparatus according to claim 1, wherein the image generator scales the calculated parameters from perfect uniformity to non-uniformity, converts the parameters to a gray scale or pseudocolor and then displays the converted parameters as the tissue uniformity image on the display means.

9. The ultrasonic diagnostic apparatus according to claim 8, wherein an ultrasonic image generated on the basis of the reflected echo signal is at least one of a B-mode image, an M-mode image and a 3D-mode image, and the image generator displays the tissue uniformity image while superposing the tissue uniformity image on the ultrasonic image.

**10.** The ultrasonic diagnostic apparatus according to claim 9, wherein the image generator subjects the tissue uniformity image to translucent processing and displays the processed tissue uniformity image while superposing the processed tissue uniformity image on the ultrasonic image.

**11.** A method by which an ultrasonic diagnostic apparatus functions, **characterized by** comprising:

a step of generating a relative frequency distribution of amplitude values at plural measurement points in a region of interest of an ultrasonic signal based on a reflected echo signal which is obtained by transmitting/receiving an ultrasonic wave to/from an examinee;
a step of calculating parameters of a probability density function having the parameters concerned, the parameters being related to uniformity of a biological tissue of the examinee, on the basis of a moment statistic quantity obtained from the generated relative frequency distribution;
a step of executing each of the steps while moving the region of interest;
a step of generating a tissue uniformity image gradated on the basis of the parameters calculated in each region of interest; and
a step of displaying the generated tissue uniformity image on display means.

**12.** The method by which the ultrasonic diagnostic apparatus functins according to claim 11, further comprising:

a step of generating a probability density function on the basis of the calculated parameters; and
a step of performing a goodness-of-fit test between the generated probability density function and the relative frequency distribution to calculate a significance probability, wherein the step of generating the gradated tissue uniformity image generates a tissue uniformity image on the basis of the parameters which make the calculated significance probability equal to or more than a set threshold value.

**13.** An ultrasonic diagnostic program making computer execute:

a procedure of generating a relative frequency distribution of amplitude values at plural measurement points in a region of interest of an ultrasonic signal based on a reflected echo signal which is obtained by transmitting/receiving an ultrasonic wave to/from an examinee;
a procedure of calculating parameters of a probability density function having the parameters concerned, the parameters being related to uniformity of a biological tissue of the examinee, on the basis of a moment statistic quantity obtained from the generated relative frequency distribution;
a procedure of executing each of the steps while moving the region of interest;
a procedure of generating a tissue uniformity image gradated on the basis of the parameters calculated in each region of interest; and
a procedure of displaying the generated tissue uniformity image on display means.

**14.** The ultrasonic diagnostic program according to claim 13, further comprising:

a procedure of generating a probability density function on the basis of the calculated parameters; and
a procedure of performing a goodness-of-fit test between the generated probability density function and the relative frequency distribution to calculate a significance probability, wherein the procedure of generating the gradated tissue uniformity image generates a tissue uniformity image on the basis of the parameters which make the calculated significance probability equal to or more than a set threshold value.

# FIG. 1

<u>10</u>

| 12 | 14 | 16 | 22 | 30 |
|---|---|---|---|---|
| ULTRASONIC PROBE | RF SIGNAL GENERATOR | ULTRASONIC IMAGE GENERATOR | DISPLAY IMAGE GENERATOR | DISPLAY UNIT |

18a

18 — CALCULATOR

UNIFORMITY CALCULATOR

20

UNIFORMITY IMAGE GENERATOR

SIGNIFICANCE PROBABILITY CALCULATOR

18b

24 — CONTROLLER

OPERATION UNIT

26

STORAGE DEVICE

28

EP 2 208 465 A1

FIG. 2

START

S10 — CREATE RELATIVE FREQUENCY DISTRIBUTION FROM AMPLITUDE IN ROI

S11 — CALCULATE PARAMETERS OF PROBABILITY DENSITY FUNCTION

S12 — CREATE PROBABILITY DENSITY FUNCTION USING CALCULATED PARAMETERS

S13 — GOODNESS-OF-FIT TEST BETWEEN RELATIVE FREQUENCY DISTRIBUTION AND ESTIMATED PROBABILITY DENSITY FUNCTION

S14 — NEXT ROI ?
Yes → S15 — MOVE ROI POSITION
No ↓

S16 — IMAGE CALCULATED PARAMETERS

S17 — SUPERPOSED DISPLAY ON B IMAGE

S18 — NEXT FRAME ?
Yes
No → END

# FIG. 3

x

y

RF SIGNAL

ROI

PARAMETER CALCULATION AND TEST

AMPLITUDE DATA FOR B IMAGE

UNIFORMITY
PARAMETER

SIGNIFICANCE
PROBABILITY

SCAN CONVERSION +
SUPERPOSED DISPLAY

x

y

SCREENING USING
THRESHOLD VALUE
BASED ON SIGNIFICANT
LEVEL

B + TISSUE UNIFORMITY IMAGE

EP 2 208 465 A1

# FIG. 4

PROBABILITY DENSITY FUNCTION BASED ON
CALCULATED PARAMETERS

ACTUALLY MEASURED RELATIVE
FREQUENCY DISTRIBUTION

PROBABILITY

AMPLITUDE

FIG. 5

FIG. 6

FIG. 7

START

S20 — CREATE RELATIVE FREQUENCY DISTRIBUTION FROM AMPLITUDE IN ROI

S21 — CALCULATE PARAMETERS OF PROBABILITY DENSITY FUNCTION

S22 — CREATE PROBABILITY DENSITY FUNCTION USING CALCULATED PARAMETERS

S23 — GOODNESS-OF-FIT TEST BETWEEN RELATIVE FREQUENCY DISTRIBUTION AND ESTIMATED PROBABILITY DENSITY FUNCTION

S24 — NEXT ROI ? — Yes

S25 — MOVE ROI POSITION

No

S26 — IMAGE CALCULATED PARAMETERS

S27 — SUPERPOSED DISPLAY ON M IMAGE

S28 — NEXT BEAM ? — Yes

No

END

EP 2 208 465 A1

# FIG. 8

RF SIGNAL

AMPLITUDE DATA FOR M IMAGE

ROI

y

PARAMETER CALCULATION AND TEST

UNIFORMITY
PARAMETER

SIGNIFICANCE
PROBABILITY

t

SCAN CONVERSION +
SUPERPOSED DISPLAY

y

SCREENING USING
THRESHOLD VALUE
BASED ON SIGNIFICANT
LEVEL

M + TISSUE UNIFORMITY IMAGE

# FIG. 9

START

S30 — CREATE RELATIVE FREQUENCY DISTRIBUTION FROM AMPLITUDE IN ROI

S31 — CALCULATE PARAMETERS OF PROBABILITY DENSITY FUNCTION

S32 — CREATE PROBABILITY DENSITY FUNCTION USING CALCULATED PARAMETERS

S33 — GOODNESS-OF-FIT TEST BETWEEN RELATIVE FREQUENCY DISTRIBUTION AND ESTIMATED PROBABILITY DENSITY FUNCTION

S34 — NEXT ROI ?

Yes → S35 — MOVE ROI POSITION

No ↓

S36 — IMAGE CALCULATED PARAMETERS

S37 — SUPERPOSED DISPLAY ON 3D IMAGE

S38 — NEXT VOLUME ?

Yes

No ↓

END

EP 2 208 465 A1

FIG. 10

RF SIGNAL

AMPLITUDE DATA FOR 3D IMAGE

ROI

PARAMETER CALCULATION AND TEST

UNIFORMITY PARAMETERS

SIGNIFICANCE PROBABILITY

SCAN CONVERSION + SUPERPOSED DISPLAY

3D + TISSUE UNIFORMITY IMAGE

SCREENING USING THRESHOLD VALUE BASED ON SIGNIFICANT LEVEL

FIG. 11

UNIFORMITY :
(1) 4.5
(2) 6.3
(3) 3.8

# FIG. 12

HEART INFARCTION SITE

EP 2 208 465 A1

## FIG. 13

HEART INFARCTION SITE

EP 2 208 465 A1

# FIG. 14

LIVER TUMOR

EP 2 208 465 A1

FIG. 15

BREAST TUMOR

| INTERNATIONAL SEARCH REPORT | | International application No. |
| --- | --- | --- |
| | | PCT/JP2008/069507 |

**A. CLASSIFICATION OF SUBJECT MATTER**
*A61B8/08*(2006.01)i

According to International Patent Classification (IPC) or to both national classification and IPC

**B. FIELDS SEARCHED**

Minimum documentation searched (classification system followed by classification symbols)
A61B8/08

Documentation searched other than minimum documentation to the extent that such documents are included in the fields searched

| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y<br>A | JP 2002-233527 A  (Aloka Co., Ltd.),<br>20 August, 2002 (20.08.02),<br>Par. Nos. [0035] to [0037], [0040], [0041],<br>[0043], [0044], [0047] to [0051], [0057],<br>[0059], [0060]; Figs. 2, 3<br>(Family: none) | 1,2,4-9,11,<br>13<br>10<br>3,12,14 |
| A | Tomoaki NAGANO et al., "Iyo Choonpa Gazo ni<br>Okeru Nyusen Shuyo no Soshikiseijo to Keitaiteki<br>Tokucho no Chushutsu", The Transactions of the<br>Institute of Electronics, Information and<br>Communication Engineers A, 2003.11, Vol.J86-A,<br>No.11, pages 1108 to 1115 | 2,11,13 |

| ☒ Further documents are listed in the continuation of Box C. | | ☐ See patent family annex. |
| --- | --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>09 January, 2009 (09.01.09) | Date of mailing of the international search report<br>27 January, 2009 (27.01.09) |
| --- | --- |
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

International application No.

PCT/JP2008/069507

C (Continuation). DOCUMENTS CONSIDERED TO BE RELEVANT

| Category* | Citation of document, with indication, where appropriate, of the relevant passages | Relevant to claim No. |
|---|---|---|
| Y | WO 1997/034530 A1 (Furuno Electric Co., Ltd.), 25 September, 1997 (25.09.97), Abstract & US 5931784 A & CN 1182357 A | 10 |
| A | Vinayak Dutt et al, K Distribution Model of Ultrasound Speckle: Fractional order SNRs and Log Compression Variance, Proceedings of 1995 IEEE Ultrasonics Symposium, 1995.11, vol.2, pp.1375-1378 | 1-14 |
| A | Torbørn Eltoft, Modeling the Amplitude Statistics of Ultrasonic Images, IEEE TRANSACTIONS ON MEDICAL IMAGING, 2006.02, vol. 25, no. 2, pp.229-240 | 1-14 |
| A | Zhong Tao et al, Evaluation of Four Probability Distribution Models for Speckle in Clinical Cardiac Ultrasound Images, IEEE TRANSACTIONS ON MEDICAL IMAGING, 2006.11, vol. 25, no. 11, pp.1483-1491 | 1-14 |
| A | JP 2006-296495 A (Toshiba Corp.), 02 November, 2006 (02.11.06), Par. Nos. [0034] to [0040] (Family: none) | 1-14 |

Form PCT/ISA/210 (continuation of second sheet) (April 2007)

**EP 2 208 465 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003061964 A

**Non-patent literature cited in the description**

- **Dutt V ; Greenleaf JF.K.** *IEEE Proc. Ultrasonics Symposium,* 1995, vol. 2, 1375-1378